# EUROPEAN PATENT SPECIFICATION

(11) **EP 3 489 771 B1**
(45) Date of publication and mention of the grant of the patent: **26.02.2020**
(21) Application number: 18176278.2
(22) Date of filing: 06.06.2018
(51) Int. Cl.: G04G 9/00, G04C 17/00

(54) **WATCH-TYPE MOBILE TERMINAL**
MOBILES ENDGERÄT VOM UHRENTYP
TERMINAL MOBILE DE TYPE MONTRE

(30) Priority: 22.11.2017 KR 20170156792
(43) Date of publication of application: 29.05.2019
(73) Proprietor: LG Electronics Inc., Yeongdeungpo-gu Seoul 07336 (KR)
(72) Inventor: KANG, Youngjin, 06772 Seoul (KR); KIM, Daeun, 06772 Seoul (KR); KIM, Hyeongjin, 06772 Seoul (KR); PARK, Dongcheon, 06772 Seoul (KR); YOO, Mijun, 06772 Seoul (KR); LEE, Byoungnam, 06772 Seoul (KR); LEE, Junghoon, 06772 Seoul (KR)
(74) Representative: Vossius & Partner Patentanwälte Rechtsanwälte mbB

(56) References cited:
- EP-A2- 3 101 882
- US-A- 5 528 559
- US-A1- 2015 131 412

## Description

### CROSS-REFERENCE TO RELATED APPLICATIONS

This application claims priority under 35 U.S.C. 119 and 365 to Korean Patent Application No. 10-2017-0156792, filed on November 22, 2017 in the Korean Intellectual Property Office.

### BACKGROUND

The present disclosure relates to a watch-type mobile terminal capable of providing more improved convenience for a user when the user uses the terminal.

Terminals may be generally classified as mobile/portable terminals or stationary terminals according to their mobility. Mobile terminals may also be classified as handheld terminals or vehicle mounted terminals according to whether or not a user may directly carry the terminal.

Mobile terminals have become increasingly more functional. Examples of such functions include data and voice communications, capturing images and video via a camera, recording audio, playing music files via a speaker system, and displaying images and video on a display. Some mobile terminals include additional functionality which supports game playing, while other terminals are configured as multimedia players. More recently, mobile terminals have been configured to receive broadcast and multicast signals which permit viewing of content such as videos and television programs.

Users mostly hold and use the mobile terminals by their hands and furthermore, the mobile terminals may be expanded to wearable devices which may be worn on their bodies. The wearable devices include a watch-type mobile terminal, a glass-type mobile terminal, a head mounted display (HMD), and so on.

Among others, the watch-type mobile terminal is formed by the adding of electronic, communication and multimedia functions to a watch which a human being always wear, and it seems that a big market is formed in the future because the watch-type mobile terminal does not provide antipathy to the human being.

Thus, research, development and commercialization of the watch-type mobile terminal are being actively conducted. US 5 528 559 A discloses a multiple display timepiece.

EP 3 101 882 A2 discloses a display device and a control method thereof.

US 2015/131412 A1 discloses a timepiece with display devices.

### SUMMARY

The present disclosure provides a watch-type mobile terminal enabling an hour hand a minute hand to serve as a second hand in a digital clock mode.

The present invention provides a watch-type mobile terminal capable of controlling the movements of an hour hand and a minute hand according to the wearing state of a watch-type mobile terminal.

According to the present invention there is provided a watch-type mobile terminal according to claim 1. Preferred embodiments are defined in dependent claims 2-11.

According to various embodiments of the present disclosure, in the digital clock mode, the physical hour hand and the physical minute hand may be effectively used. In addition, the hour hand and the minute hand are moved in the state that the hour hand and the minute hand are overlapped with each other, thereby preventing information from being hidden on the display unit.

### BRIEF DESCRIPTION OF THE DRAWINGS

FIG. 1 is a block diagram illustrating a mobile terminal related to the present invention.
FIG. 2 is a perspective view illustrating one example of a watch-type mobile terminal related to the present invention.
FIG. 3 is a flowchart illustrating an operating method of a watch-type mobile terminal according to an embodiment of the present disclosure.
FIGS. 4A and 4B are views illustrating the movements of the hour hand and the minute hand depending on the clock modes of the watch-type mobile terminal according to an embodiment of the present disclosure.
FIG. 5 is a flowchart illustrating the operating method of the watch-type mobile terminal according to another embodiment of the present invention.
FIGS. 6A to 6C are views illustrating that the movements of the hour hand and the minute hand is controlled as the screen of the display unit is turned off, when the watch-type mobile terminal is put on the user, according to an embodiment of the present disclosure.
FIGS. 7A to 7C are views illustrating that the movements of the hour hand and the minute hand is controlled as the screen of the display unit is turned off, when the watch-type mobile terminal is not put on the user, according to an embodiment of the present disclosure.

### DETAILED DESCRIPTION OF THE EMBODIMENTS

Hereinafter, embodiments are described in more detail with reference to accompanying drawings and regardless of the drawings symbols, same or similar components are assigned with the same reference numerals and thus repetitive for those are omitted. Since the suffixes "module" and "unit" for components used in the following description are given and interchanged for easiness in making the present disclosure, they do not have distinct meanings or functions. In the following description, detailed descriptions of well-known functions or constructions will be omitted because they would obscure the present invention in unnecessary detail. Also, the accompanying drawings are used to help easily understanding embodiments disclosed herein but the technical idea of the present invention is not limited thereto. It should be understood that all of variations, equivalents or substitutes contained in the concept and technical scope of the present invention are also included.

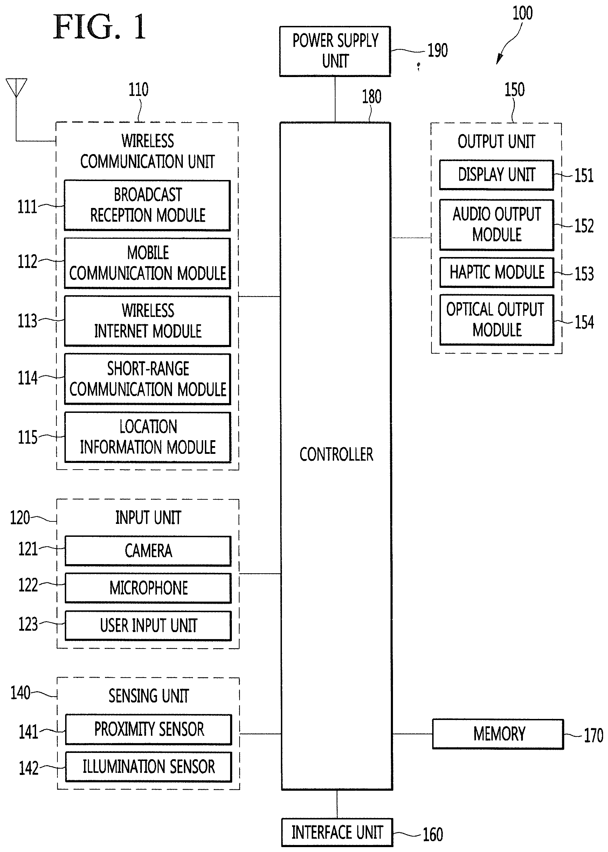

FIG. 1 is a block diagram for explaining a mobile terminal related to an embodiment.

A watch-type mobile terminal 100 may include a wireless communication unit 110, an input unit 120, a sensing unit 140, an output unit 150, an interface unit 160, a memory 170, a control unit 180, and a power supply unit 190. In implementing the watch-type mobile terminal, components shown in FIG. 1 are not necessary, so the watch-type mobile terminal described in this specification may include more or less components than those listed above.

In more detail, the wireless communication unit 110 typically includes one or more modules which permit communications such as wireless communications between the watch-type mobile terminal 100 and a wireless communication system, communications between the watch-type mobile terminal 100 and another mobile terminal, communications between the watch-type mobile terminal 100 and an external server. Further, the wireless communication unit 110 typically includes one or more modules which connect the watch-type mobile terminal 100 to one or more networks.

The wireless communication unit 110 includes one or more of a broadcast receiving module 111, a mobile communication module 112, a wireless Internet module 113, a short-range communication module 114, and a location information module 115.

The input unit 120 includes a camera 121 for obtaining images or video, a microphone 122, which is one type of audio input device for inputting an audio signal, and a user input unit 123 (for example, a touch key, a push key, a mechanical key, a soft key, and the like) for allowing a user to input information. Data (for example, audio, video, image, and the like) is obtained by the input unit 120 and may be analyzed and processed by control unit 180 according to device parameters, user commands, and combinations thereof.

The sensing unit 140 may include one or more sensors to sense at least one of internal information of the watch-type mobile terminal, surrounding environment information of the watch-type mobile terminal, and user information. For example, the sensing unit 140 may include at least one of a proximity sensor 141, an illumination sensor 142, a touch sensor, an acceleration sensor, a magnetic sensor, a G-sensor, a gyroscope sensor, a motion sensor, an RGB sensor, an infrared (IR) sensor, a finger scan sensor, a ultrasonic sensor, an optical sensor (for example, camera 121), a microphone 122, a battery gauge, an environment sensor (for example, a barometer, a hygrometer, a thermometer, a radiation detection sensor, a thermal sensor, and a gas sensor, among others), and a chemical sensor (for example, an electronic nose, a health care sensor, a biometric sensor, and the like). The watch-type mobile terminal 100 may be configured to utilize the combination of information sensed of at least two of the sensors.

The output unit 150 is typically configured to output various types of information, such as audio, video, tactile output, and the like. The output unit 150 is shown having a display unit 151, an audio output module 152, a haptic module 153, and an optical output module 154. The display unit 151 may have an inter-layered structure or an integrated structure with a touch sensor in order to facilitate a touch screen. The touch screen may provide an output interface between the watch-type mobile terminal 100 and a user, as well as function as the user input unit 123 which provides an input interface between the watch-type mobile terminal 100 and the user.

The interface unit 160 serves as an interface with various types of external devices that may be coupled to the watch-type mobile terminal 100. The interface unit 160, for example, may include any of wired or wireless ports, external charging ports, wired or wireless data ports, memory card ports, ports for connecting a device having an identification module, audio input/output (I/O) ports, video I/O ports, earphone ports, and the like. In some cases, the watch-type mobile terminal 100 may perform assorted control functions associated with a connected external device, in response to the external device being connected to the interface unit 160.

The memory 170 is typically implemented to store data to support various functions or features of the watch-type mobile terminal 100. For instance, the memory 170 may be configured to store application programs executed in the watch-type mobile terminal 100, data or instructions for operations of the watch-type mobile terminal 100, and the like. Some of these application programs may be downloaded from an external server via wireless communication. Other application programs may be installed within the watch-type mobile terminal 100 at time of manufacturing or shipping, which is typically the case for basic functions of the watch-type mobile terminal 100 (for example, receiving a call, placing a call, receiving a message, sending a message, and the like). It is common for application programs to be stored in the memory 170, installed in the watch-type mobile terminal 100, and executed by the control unit 180 to perform an operation (or function) for the watch-type mobile terminal 100.

The control unit 180 typically functions to control overall operation of the watch-type mobile terminal 100, in addition to the operations associated with the application programs. The control unit 180 may provide or process information or functions appropriate for a user by processing signals, data, information and the like, which are input or output by the various components as described above, or activating application programs stored in the memory 170.

The control unit 180 may provide or process information or functions appropriate for a user by processing signals, data, information and the like, which are input or output by the various components depicted in FIG. 1, or activating application programs stored in the memory 170. Further, the control unit 180 operates at least two of components included in the watch-type mobile terminal 100 by combining the at least two of components.

The power supply unit 190 may be configured to receive external power or provide internal power in order to supply appropriate power required for operating elements and components included in the watch-type mobile terminal 100, under the control of the control unit 180. The power supply unit 190 may include a battery, and the battery may be configured to be embedded in the terminal body, or configured to be detachable from the terminal body.

At least some of the components may operate in cooperation with each other to realize the operation, the control, or the control method of a watch-type mobile terminal according to various embodiments to be described below. In addition, the operation, the control, or the control method of a watch-type mobile terminal may be realized on the watch-type mobile terminal by running at least one application program stored in the memory 170.

Before the description of various embodiments realized through the watch-type mobile terminal 100 described above, the above components will be described in more detail with reference to FIG. 1.

Regarding the wireless communication unit 110, the broadcast receiving module 111 of the wireless communication unit is typically configured to receive a broadcast signal and/or broadcast associated information from an external broadcast managing entity via a broadcast channel. The broadcast channel may include a satellite channel, a terrestrial channel, or both. In some embodiments, two or more broadcast receiving modules may be provided in the watch-type mobile terminal 100 for simultaneously receiving of two or more broadcast channels, or to support switching among broadcast channels.

The mobile communication module 112 may transmit and/or receive wireless signals to and from one or more network entities. Typical examples of a network entity include a base station, an external mobile terminal, a server, and the like. Such network entities form part of a mobile communication network, which is constructed according to technical standards or communication methods for mobile communications (for example, Global System for Mobile Communication (GSM), Code Division Multi Access (CDMA), CDMA2000(Code Division Multi Access 2000), EV-DO(Enhanced Voice-Data Optimized or Enhanced Voice-Data Only), Wideband CDMA (WCDMA), High Speed Downlink Packet access (HSDPA), HSUPA(High Speed Uplink Packet Access), Long Term Evolution (LTE), LTE-A(Long Term Evolution-Advanced), and the like). Examples of wireless signals transmitted and/or received via the mobile communication module 112 include audio call signals, video (telephony) call signals, or various formats of data to support communication of text and multimedia messages.

Examples of wireless signals include voice call signals, video (telephony) call signals, or various formats of data to support communication of text and multimedia messages.

The wireless Internet module 113, which refers to a module to access the wireless Internet, may be embedded in the mobile terminal 100 or provided outside the mobile terminal 100. The wireless Internet module 113 may transmit and/or receive wireless signals via communication networks according to wireless Internet technologies

Examples of such wireless Internet access include Wireless LAN (WLAN), Wireless Fidelity (Wi-Fi), Wi-Fi Direct, Digital Living Network Alliance (DLNA), Wireless Broadband (WiBro), Worldwide Interoperability for Microwave Access (WiMAX), High Speed Downlink Packet Access (HSDPA), HSUPA(High Speed Uplink Packet Access), Long Term Evolution (LTE), LTE-A(Long Term Evolution-Advanced), and the like. The wireless Internet module 113 may transmit/receive data according to one or more of such wireless Internet technologies, and other Internet technologies as well.

When the wireless Internet access is implemented according to, for example, WiBro, HSDPA,HSUPA, GSM, CDMA, WCDMA, LTE, LTE-A and the like, as part of a mobile communication network, the wireless Internet module 113 performs such wireless Internet access. As such, the Internet module 113 may cooperate with, or function as, the mobile communication module 112.

The short-range communication module 114 is configured to facilitate short-range communications. Suitable technologies for implementing such short-range communications include Bluetooth™, Radio Frequency Identification (RFID), Infrared Data Association (IrDA), Ultra-WideBand (UWB), ZigBee, Near Field Communication (NFC), Wireless-Fidelity (Wi-Fi), Wi-Fi Direct, Wireless USB(Wireless Universal Serial Bus), and the like. The short-range communication module 114 in general supports wireless communications between the watch-type mobile terminal 100 and a wireless communication system, communications between the mobile terminal 100 and another mobile terminal 100, or communications between the mobile terminal and a network where another mobile terminal 100 (or an external server) is located, via wireless area networks. One example of the wireless area networks is a wireless personal area networks.

The short-range communication module 114 may sense or recognize the wearable device, and permit communication between the wearable device and the watch-type mobile terminal 100. In addition, when the sensed wearable device is a device which is authenticated to communicate with the watch-type mobile terminal 100, the control unit 180, for example, may cause transmission of data processed in the watch-type mobile terminal 100 to the wearable device via the short-range communication module 114. Hence, a user of the wearable device may use the data processed in the watch-type mobile terminal 100 on the wearable device. For example, when a call is received in the watch-type mobile terminal 100, the user may answer the call using the wearable device. Also, when a message is received in the watch-type mobile terminal 100, the user may check the received message using the wearable device.

The location information module 115 is a module to acquire a location of the mobile terminal. As an example, the location information module 115 representatively includes a Global Position System (GPS) module, a Wi-Fi module, or both. For example, if the mobile terminal utilizes the GPS module, the position of the mobile terminal may be acquired by using the signal sent from the GPS satellite. Alternatively, if the mobile terminal utilizes the Wi-Fi module, the position of the mobile terminal may be acquired based on the information of a wireless access point (AP) for transmitting or receiving a wireless signal together with the Wi-Fi module. If necessary, the location information module 115 may any one of functions of other modules of the wireless communication unit 110 to alternatively or additionally acquire the location data of the mobile terminal. The location information module 115, which is a module used to acquire the location (or current location) of the mobile terminal, is not limited to a module to directly calculate or acquire the position of the mobile terminal.

The input unit 120 may be configured to permit various types of input to the mobile terminal 120. Examples of such input include audio, image, video, data, and user input. In order to obtain the input of video information, the watch-type mobile terminal 100 may include one or more cameras 121. Such cameras 121 may process image frames of still pictures or video obtained by image sensors in a video or image capture mode. The processed image frames may be displayed on the display unit 151 or stored in memory 170. In some cases, the cameras 121 may be arranged in a matrix configuration to permit a plurality of images having various angles or focal points to be input to the watch-type mobile terminal 100. As another example, the cameras 121 may be located in a stereoscopic arrangement to acquire left and right images for implementing a stereoscopic image.

The microphone 122 converts external sound signal into electrical voice signal. The processed voice data may be utilized variously depending on functions (or application under running) executed by the watch-type mobile terminal 100. If desired, the microphone 122 may include assorted noise removing algorithms to remove unwanted noise generated in the course of receiving the external sound.

The user input unit 123 is a component that permits input by a user. Such user input may enable the control unit 180 to control operation of the watch-type mobile terminal 100. The user input unit 123 may include one or more of a mechanical input element (for example, a key, a button located on a front and/or rear surface or a side surface of the watch-type mobile terminal 100, a dome switch, a jog wheel, a jog switch, and the like), or a touch-sensitive input, among others. As one example, the touch-sensitive input may be a virtual key or a soft key, which is displayed on a touch screen through software processing, or a touch key which is located on the mobile terminal at a location that is other than the touch screen. On the other hand, the virtual key or the visual key may be displayed on the touch screen in various shapes, for example, graphic, text, icon, video, or a combination thereof.

The sensing unit 140 is generally configured to sense one or more of internal information of the mobile terminal, surrounding environment information of the mobile terminal, user information, or the like. The control unit 180 generally cooperates with the sending unit 140 to control operation of the watch-type mobile terminal 100 or execute data processing, a function or an operation associated with an application program installed in the mobile terminal based on the sensing provided by the sensing unit 140. The sensing unit 140 may be implemented using any of a variety of sensors, some of which will now be described in more detail.

The proximity sensor 141 may include a sensor to sense presence or absence of an object approaching a surface, or an object located near a surface, by using an electromagnetic field, infrared rays, or the like without a mechanical contact. The proximity sensor 141 may be arranged at an inner region of the mobile terminal covered by the touch screen, or near the touch screen.

The proximity sensor 141, for example, may include any of a transmissive type photoelectric sensor, a direct reflective type photoelectric sensor, a mirror reflective type photoelectric sensor, a high-frequency oscillation proximity sensor, a capacitance type proximity sensor, a magnetic type proximity sensor, an infrared rays proximity sensor, and the like. When the touch screen is implemented as a capacitance type, the proximity sensor 141 may sense proximity of a pointer relative to the touch screen by changes of an electromagnetic field, which is responsive to an approach of an object with conductivity. In this case, the touch screen (touch sensor) may also be categorized as a proximity sensor.

The term "proximity touch" will often be referred to herein to denote the scenario in which a pointer is positioned to be proximate to the touch screen without contacting the touch screen. The term "contact touch" will often be referred to herein to denote the scenario in which a pointer makes physical contact with the touch screen. For the position corresponding to the proximity touch of the pointer relative to the touch screen, such position will correspond to a position where the pointer is perpendicular to the touch screen. The proximity sensor 141 may sense proximity touch, and proximity touch patterns (for example, distance, direction, speed, time, position, moving status, and the like). In general, the control unit 180 processes data (or information) corresponding to proximity touches and proximity touch patterns sensed by the proximity sensor 141, and cause output of visual information on the touch screen. In addition, the control unit 180 may control the watch-type mobile terminal 100 to execute different operations or process different data according to whether a touch with respect to a point on the touch screen is either a proximity touch or a contact touch.

A touch sensor may sense a touch applied to the touch screen, such as display unit 151, using any of a variety of touch methods. Examples of such touch methods include a resistive type, a capacitive type, an infrared type, and a magnetic field type, among others.

As one example, the touch sensor may be configured to convert changes of pressure applied to a specific part of the display unit 151, or convert capacitance occurring at a specific part of the display unit 151, into electric input signals. The touch sensor may also be configured to sense not only a touched position and a touched area, but also touch pressure and/or touch capacitance. A touch object is generally used to apply a touch input to the touch sensor. Examples of typical touch objects include a finger, a touch pen, a stylus pen, a pointer, or the like.

When a touch input is sensed by a touch sensor, a corresponding signal (signals) may be transmitted to a touch controller. The touch controller may process the received signal (signals), and then transmit corresponding data to the control unit 180. Accordingly, the control unit 180 may sense which region of the display unit 151 has been touched. Here, the touch controller may be a component separate from the control unit 180, the control unit 180, and combinations thereof.

Meanwhile, the control unit 180 may execute the same or different controls according to a type of touch object that touches the touch screen or a touch key provided in addition to the touch screen. Whether to execute the same or different control according to the object which provides a touch input may be decided based on a current operating state of the watch-type mobile terminal 100 or a currently executed application program, for example.

Meanwhile, the touch sensor and the proximity sensor may be implemented individually, or in combination, to sense various types of touches. Such touches includes a short (or tap) touch, a long touch, a multi-touch, a drag touch, a flick touch, a pinch-in touch, a pinch-out touch, a swipe touch, a hovering touch, and the like.

An ultrasonic sensor may be implemented to recognize position information relating to a touch object using ultrasonic waves. The control unit 180, for example, may calculate a position of a wave generation source based on information sensed by an illumination sensor and a plurality of ultrasonic sensors. Since light is much faster than ultrasonic waves, the time for which the light reaches the optical sensor is much shorter than the time for which the ultrasonic wave reaches the ultrasonic sensor. The position of the wave generation source may be calculated using this fact. For instance, the position of the wave generation source may be calculated using the time difference from the time that the ultrasonic wave reaches the sensor based on the light as a reference signal.

The camera 121, which is described as an element of the input unit 12, typically includes at least one a camera sensor (CCD, CMOS etc.), a photo sensor (or image sensors), and a laser sensor.

Implementing the camera 121 with a laser sensor may allow detection of a touch of a physical object with respect to a 3D stereoscopic image. The photo sensor may be laminated on, or overlapped with, the display device. The photo sensor may be configured to scan the movement of the physical object in proximity to the touch screen. In more detail, the photo sensor may include photo diodes and transistors at rows and columns to scan content received at the photo sensor using an electrical signal which changes according to the quantity of applied light. Namely, the photo sensor may calculate the coordinates of the physical object according to variation of light to thus obtain position information of the physical object.

The display unit 151 is generally configured to output information processed in the watch-type mobile terminal 100. For example, the display unit 151 may display execution screen information of an application program executing at the watch-type mobile terminal 100 or user interface (UI) and graphic user interface (GUI) information in response to the execution screen information.

The audio output module 152 is generally configured to output audio data received from the wireless communication unit 110 or stored in the memory 170 during modes such as a signal reception mode, a call mode, a record mode, a voice recognition mode, a broadcast reception mode, and the like. The audio output module 152 may provide audible output related to a particular function (e.g., a call signal reception sound, a message reception sound, etc.) performed by the watch-type mobile terminal 100. The audio output module 152 may also be implemented as a receiver, a speaker, a buzzer, or the like.

A haptic module 153 may be configured to generate various tactile effects that a user feels, perceive, or otherwise experience. A typical example of a tactile effect generated by the haptic module 153 is vibration. The strength, pattern and the like of the vibration generated by the haptic module 153 may be controlled by user selection or setting by the controller. For example, the haptic module 153 may output different vibrations in a combining manner or a sequential manner.

The haptic module 153 may generate, in addition to the vibration, various other tactile effects, including an effect by stimulation such as a pin arrangement vertically moving to contact skin, a spray force or suction force of air through a jet orifice or a suction opening, a touch to the skin, a contact of an electrode, electrostatic force, an effect by reproducing the sense of cold and warmth using an element that may absorb or generate heat, and the like.

The haptic module 153 may also be implemented to allow the user to feel a tactile effect through a muscle sensation such as the user's fingers or arm, as well as transferring the tactile effect through direct contact. Two or more haptic modules 153 may be provided according to the particular configuration of the watch-type mobile terminal 100.

An optical output module 154 may output a signal for indicating an event generation using light of a light source. Examples of events generated in the watch-type mobile terminal 100 may include message reception, call signal reception, a missed call, an alarm, a schedule notice, an email reception, information reception through an application, and the like.

A signal output by the optical output module 154 may be implemented in such a manner that the watch-type mobile terminal emits monochromatic light or light with a plurality of colors to the front surface or rear surface thereof. The signal output may be terminated as the watch-type mobile terminal senses that a user has checked the generated event.

The interface unit 160 serves as an interface for external devices to be connected with the mobile terminal 100. For example, the interface unit 160 may receive data transmitted from an external device, receive power to transfer to elements and components within the watch-type mobile terminal 100, or transmit internal data of the watch-type mobile terminal 100 to such external device. The interface unit 160 may include wired or wireless headset ports, external power supply ports, wired or wireless data ports, memory card ports, ports for connecting a device having an identification module, audio input/output (I/O) ports, video I/O ports, earphone ports, or the like.

The identification module may be a chip that stores various pieces of information for authenticating authority of using the watch-type mobile terminal 100 and may include a user identity module (UIM), a subscriber identity module (SIM), a universal subscriber identity module (USIM), and the like. In addition, the device having the identification module (also referred to herein as an "identifying device") may take the form of a smart card. Accordingly, the identifying device may be connected with the terminal 100 via the interface unit 160.

When the watch-type mobile terminal 100 is connected with an external cradle, the interface unit 160 may serve as a passage to allow power from the cradle to be supplied to the watch-type mobile terminal 100 or may serve as a passage to allow various command signals input by the user from the cradle to be transferred to the mobile terminal there through. Various command signals or power input from the cradle may operate as signals for recognizing that the mobile terminal is properly mounted on the cradle.

The memory 170 may store programs to support operations of the control unit 180 and store input/output data (for example, phonebook, messages, still images, videos, etc.). The memory 170 may store data related to various patterns of vibrations and audio which are output in response to touch inputs on the touch screen.

The memory 170 may include one or more types of storage mediums including a Flash memory, a hard disk, a solid state disk, a silicon disk, a multimedia card micro type, a card-type memory (e.g., SD or DX memory, etc.), a Random Access Memory (RAM), a Static Random Access Memory (SRAM), a Read-Only Memory (ROM), an Electrically Erasable Programmable Read-Only Memory (EEPROM), a Programmable Read-Only memory (PROM), a magnetic memory, a magnetic disk, an optical disk, and the like. The watch-type mobile terminal 100 may also be operated in relation to a network storage device that performs the storage function of the memory 170 over a network, such as the Internet.

Meanwhile, as described above, the control unit 180 may typically control the general operations of the watch-type mobile terminal 100. For example, the control unit 180 may set or release a lock state for restricting a user from inputting a control command with respect to applications when a status of the mobile terminal meets a preset condition.

The control unit 180 may also perform the controlling and processing associated with voice calls, data communications, video calls, and the like, or perform pattern recognition processing to recognize a handwriting input or a picture drawing input performed on the touch screen as characters or images, respectively. In addition, the control unit 180 may control one or a combination of those components in order to implement various exemplary embodiments disclosed herein.

The power supply unit 190 receives external power or provides internal power and supply the appropriate power required for operating respective elements and components included in the watch-type mobile terminal 100 under the control of the control unit 180. The power supply unit 190 may include a battery, which is typically rechargeable or be detachably coupled to the terminal body for charging.

The power supply unit 190 may include a connection port. The connection port may be configured as one example of the interface unit 160 to which an external charger for supplying power to recharge the battery is electrically connected.

As another example, the power supply unit 190 may be configured to recharge the battery in a wireless manner without use of the connection port. According to the present embodiment, the power supply unit 190 may receive power, transferred from an external wireless power transmitter, using at least one of an inductive coupling method which is based on magnetic induction or a magnetic resonance coupling method which is based on electromagnetic resonance.

Various embodiments described herein may be implemented in a computer-readable medium, a machine-readable medium, or similar medium using, for example, software, hardware, or any combination thereof.

First, the communication system may use mutually different wireless interfaces and/or physical layers. Examples of such air interfaces utilized by the communication systems include Frequency Division Multiple Access (FDMA), Time Division Multiple Access (TDMA), Code Division Multiple Access (CDMA), and Universal Mobile Telecommunications System (UMTS), the Long Term Evolution (LTE) of the UMTS, the Global System for Mobile Communications (GSM), and the like.

By way of non-limiting example only, further description will relate to a CDMA communication system, but such teachings apply equally to other system types including Orthogonal Frequency Division Multiplexing (OFDM) wireless communication as well as the CDMA wireless communication system.

A CDMA wireless communication system is shown having at least one terminal 100, at least one base station (BS) (which is named Node B or Evolved Node B), at least one of base station controllers (BSCs), and a mobile switching center (MSC). The MSC is configured to interface with a conventional Public Switch Telephone Network (PSTN). The MSC is also configured to interface with the BSCs. The BSCs are coupled to the base stations via backhaul lines. The backhaul lines may be configured in accordance with any of several known interfaces including, for example, E1/T1, ATM, IP, PPP, Frame Relay, HDSL, ADSL, or xDSL. Hence, the plurality of BSCs may be included in the CDMA wireless communication system.

Each base station may include one or more sectors, each sector having an omni-directional antenna or an antenna pointed in a particular direction radially away from the BS. Alternatively, each sector may include two or more different antennas. Each BS may be configured to support a plurality of frequency assignments, with each frequency assignment having a particular spectrum (e.g., 1.25 MHz, 5 MHz, etc.).

The intersection of sector and frequency assignment may be referred to as a CDMA channel. The BS may also be referred to as Base Station Transceiver Subsystems (BTSs). In some cases, the term "base station" may be used to refer collectively to a BSC, and one or more BS. The base stations may also be denoted as "cell sites." Alternatively, individual sectors of a specific base station may be referred to as cell sites.

A broadcasting transmitter (BT) transmits a broadcast signal to the terminals 100 operating within the system. The broadcast receiving module 111 as illustrated in FIG. 1 is typically configured inside the watch-type mobile terminal 100 to receive broadcast signals transmitted by the BT.

In addition, Global Positioning System (GPS) satellites may be connected with the CDMA wireless communication to identify the position of the watch-type mobile terminal 100. Such satellites 300 facilitate locating the position of at least one of plural watch-type mobile terminal 100. It is understood that useful position information may be obtained with greater or fewer satellites than two satellites. The position of the watch-type mobile terminal 100 may be tracked by using all technologies that is able to tract the location as well as the GPS tracking technology. In addition, at least one of the GPS satellites may alternatively or additionally be configured to provide satellite DMB transmissions.

The location information module 115 included in the mobile terminal is used for detecting, computing, or identifying the position of the mobile terminal, and may representatively include a Global Position System (GPS) module and a WiFi (Wireless Fidelity) module. If necessary, the location information module 115 may alternatively or additionally perform any of the other functions of the wireless communication unit 110 to obtain data on the position of the mobile terminal.

The GPS module 115 is able to precisely calculate current 3-dimensional position information based on at least one of longitude, latitude and altitude and direction (or orientation) by calculating distance information and precise time information from at least three satellites and then applying triangulation to the calculated information. Currently, location and time information are calculated using three satellites, and errors of the calculated location position and time information are then amended using another satellite. Besides, the GPS module 115 is able to calculate speed information by continuously calculating a real-time current location. However, it is difficult to measure the position of the mobile terminal by using the GPS module in a shadow area, such as an interior, of a satellite signal. Accordingly, in order to compensate for the positioning based on the GPS scheme, a WiFi positioning system (WPS) may be utilized.

The WiFi Positioning System (WPS) is a technology of tracking the position of the mobile terminal 100 by using a WiFi module included in the mobile terminal 100 and a wireless access point (AP) which transceives a wireless signal together the WiFi module. The WPS denotes a wireless local area network (WLAN) using WiFi

The WiFi positioning system may include a WiFi positioning server, the mobile terminal 100, a wireless AP connected with the mobile terminal 100, and a database having arbitrary wireless AP information stored therein.

The mobile terminal 100 in connection with the wireless AP may transmit a location information request message to the WiFi positioning system.

A WiFi positioning server extracts information of a wireless AP connected with the mobile terminal 100 based on a location information request message (or signal) of the mobile terminal 100. The information of the wireless AP connected with the mobile terminal 100 may be transmitted to the Wi-Fi positioning server through the mobile terminal 100 or may be transmitted from the wireless AP to the Wi-Fi location server.

The information of the wireless AP to be extracted based on the location information request message of the mobile terminal 100 includes a MAC address, an SSID (Service Set Identification), a Received Signal Strength Indicator (RSSI), a Reference Signal Received Power (RSRP), Reference Signal Received Quality (RSRQ), channel information, privacy, a network type, signal strength, and noise strength.

The WiFi positioning server may receive the information of the wireless AP connected to the mobile terminal 100 as described above, and may extract wireless AP information corresponding to the wireless AP connected to the mobile terminal from the pre-established database. The information of any wireless APs stored in the database may be information such as MAC address, SSID, channel information, Privacy, Network type, latitude and longitude coordinates of the wireless AP, the name of building at which the wireless AP is located, the floor number of the building, the detailed indoor location information (GPS coordinate available) of the building, an AP owner's address, a phone number, and the like. In this case, in order to remove wireless APs provided using a mobile AP or an illegal MAC address during a location determining process, the Wi-Fi positioning server may extract only a predetermined number of wireless AP information in order of high RSSI.

Then, the Wi-Fi positioning server may extract (analyze) location information of the mobile terminal 100 using at least one wireless AP information extracted from the database. The location information of the mobile terminal 100 may be extracted (or analyzed) by comparing the included information and the received wireless AP information.

A method for extracting (analyzing) location information of the mobile terminal 100 may include a Cell-ID method, a fingerprint method, a trigonometry method, a landmark method, and the like.

The Cell-ID method is used to determine a position of a wireless AP having the largest signal strength, among peripheral wireless AP information collected by the mobile terminal, as a position of the mobile terminal. The Cell-ID method is an implementation that is minimally complex, does not require additional costs, and location information may be rapidly acquired. However, in the Cell-ID method, the precision of positioning may fall below a desired threshold when the installation density of wireless APs is low.

The fingerprint method is used to collect signal strength information by selecting a reference position from a service area, and to track a position of a mobile terminal using the signal strength information transmitted from the mobile terminal based on the collected information. In order to use the fingerprint method, it is necessary for the characteristics of radio signals to be pre-stored in the form of a database.

The trigonometry method is used to calculate a position of a mobile terminal based on a distance between coordinates of at least three wireless APs and the mobile terminal. In order to measure the distance between the mobile terminal and the wireless APs, signal strength may be converted into distance information, Time of Arrival (ToA), Time Difference of Arrival (TDoA), Angle of Arrival (AoA), or the like may be taken for transmitted wireless signals.

The landmark method is used to measure a position of the mobile terminal using a known landmark transmitter.

In addition to these position location methods, various algorithms may be used to extract (analyze) location information of a mobile terminal.

[103] Such extracted location information of the mobile terminal 100 may be transmitted to the mobile terminal 100 through the Wi-Fi positioning server, thereby acquiring location information of the mobile terminal 100.

The mobile terminal 100 may acquire location information by being connected with at least one wireless AP. The number of wireless APs required to acquire location information of the mobile terminal 100 may be variously changed according to a wireless communication environment within which the mobile terminal 100 is positioned.

Various embodiments described herein may be implemented in a computer-readable medium, a machine-readable medium, or similar medium using, for example, software, hardware, or any combination thereof.

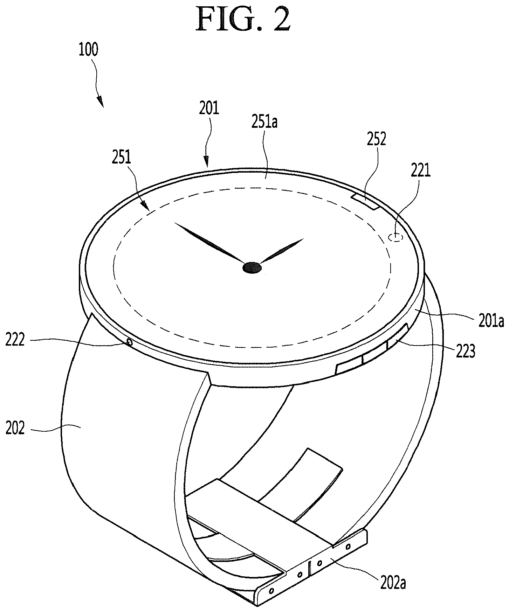

FIG. 2 is a perspective view illustrating one example of the watch-type mobile terminal related to the present invention.

The watch-type mobile terminal 100 illustrated in FIG. 2 may include all components included in FIG. 1.

The display unit 251 of the watch-type mobile terminal 100 illustrated in FIG. 2 may have a circular shape, but the present invention is not limited thereto. The display unit 251 may have an oval shape or a rectangular shape. The shape of the display unit 251 of the present invention may have various shapes sufficient to provide a visually good image for a user and to help the user manipulate the display unit 251.

Referring to FIG. 2, the watch-type mobile terminal 100 includes a main body 201 including the display unit 251 and a band 202 coupled to the main body 201 and configured to be worn on the wrist. The display unit 251 may correspond to the touch screen 151 of FIG 1.

The main body 201 includes a case that forms an outer appearance. As shown in drawing, the case may include a first case 201a and a second case 201b which define an internal space for receiving various electronic components. However, the present invention is not limited thereto, but one case is included to provide the internal space, so that the watch-type mobile terminal 100 may be realized in the form of a unibody.

The watch-type mobile terminal 100 may be configured to enable wireless communication, and the main body 201 may be equipped with an antenna for wireless communication. Meanwhile, the performance of the antenna may be enhanced through the case. For example, a case including a conductive material may be configured to electrically be connected with the antenna and thus to expand the ground or a radiation area.

The display unit 251 may be disposed on the front surface of the main body 201 to output information. The display unit 251 may include a touch sensor so that the display unit 251 may be realized as a touch screen. As illustrated in drawings, a window 251a of the display unit 251 may be mounted on the first case 201a to form a front surface of a terminal body together with the first case 201a. The case may be referred to as a bezel.

The main body 201 may include a sound output unit 252, a camera 221, a microphone 222, a user input unit 223, and the like. In the case that the display unit 251 is realized as a touch screen, the display unit 251 may serve as a user input unit 223. Accordingly, the main body 201 may not have a separate key.

The band 202 is worn on the wrist to surround a wrist, and may be formed of a flexible material for easy wearing. For example, the band 202 may be formed of leather, rubber, silicone, synthetic resin material, or the like. In addition, the band 210 is provided detachably from the main body 201. Accordingly, the band 202 may be replaceable with various shapes of bands according to the preferences of a user.

Meanwhile, the band 202 may be used to expand the performance of the antenna. For example, the band 202 may include a ground extension part (not illustrated) that is electrically connected to the antenna to expand the ground area.

The band 202 may include a fastener 202a. The fastener 202a may be by a buckle, a snap-fit hook structure, or Velcro (trademark), and may include a section having elasticity or elastic material. The present drawing illustrates an example that the fastener 202a is realized in the shape of a buckle.

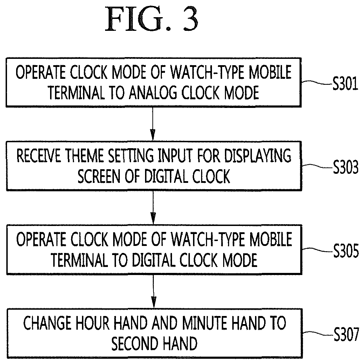

FIG. 3 is a flowchart illustrating an operating method of a watch-type mobile terminal according to an embodiment of the present disclosure.

According to an embodiment of the present disclosure, a clock mode of a watch-type mobile terminal 100 may include a digital clock mode and an analog clock mode.

The digital clock mode may be a mode that time is expressed using digital numbers on the display unit 251.

The analog clock mode may be a mode that time is expressed using a physical hour hand and a physical minute hand positioned on the display unit 251.

Referring to FIG. 3, the control unit 180 of the watch-type mobile terminal 100 operates the clock mode of the watch-type mobile terminal 100 to the analog clock mode (S301).

In the analog clock mode, the control unit 180 may control the operations of the hour hand and the minute hand such that the hour hand and the minute hand positioned on the display unit 251 represent current time.

The control unit 180 receives a theme setting input for displaying the screen of the digital clock (S303), and operates the clock mode of the watch-type mobile terminal 100 to the digital clock mode, in response to the received setting input (S305).

In this case, the theme setting input for displaying the screen of the digital clock may be an input for switching the clock mode of the watch-type mobile terminal 100 to the digital clock mode.

The control unit 180 changes the hour hand and the minute hand positioned on the display unit 251 to a second hand, in the digital clock mode (S307).

According to an embodiment, the control unit 180 may control the hour hand and the minute hand to be overlapped with each other when receiving the setting input. In other words, the control unit 180 may make the hour hand and the minute hand overlapped with each other to be aligned in a line with each other.

The control unit 180 may control the hour hand and the minute hand to be overlapped with each other. The control unit 180 may control the movements of the hour hand and the minute hand such that the overlapped hour hand and minute hand serve as a second hand.

The details of FIG. 3 will be described with reference to following drawings.

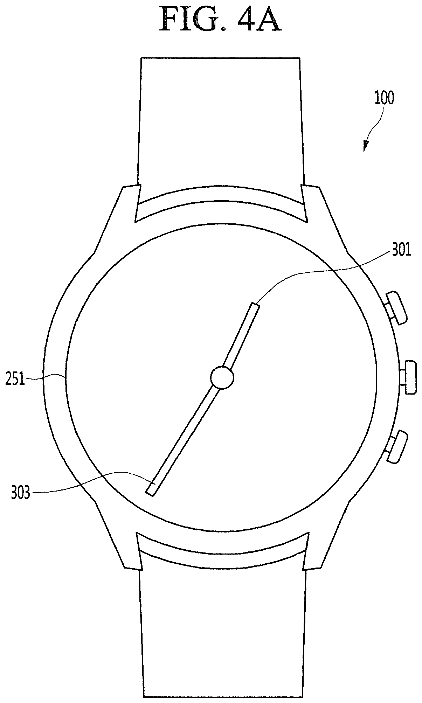

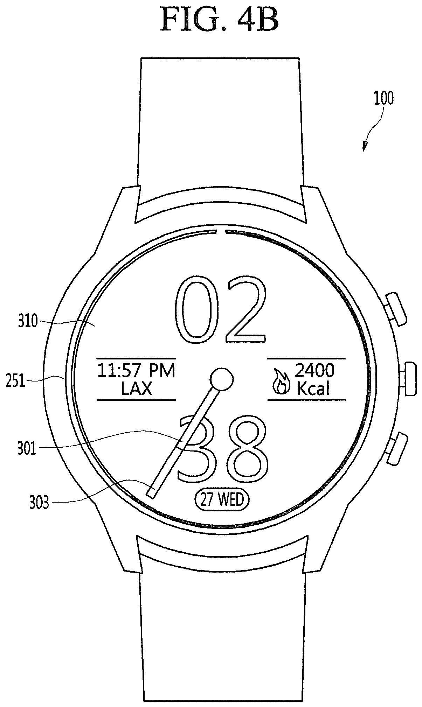

FIGS. 4A and 4B are views illustrating the movements of the hour hand and the minute hand depending on the clock modes of the watch-type mobile terminal according to an embodiment of the present disclosure.

FIG. 4A is a front view of the watch-type mobile terminal 100 when the clock mode of the watch-type mobile terminal 100 operates to the analog clock mode. FIG. 4B is a front view of the watch-type mobile terminal 100 when the clock mode of the watch-type mobile terminal 100 operates to the digital clock mode.

Referring to FIG. 4A, a physical hour hand 301 and a physical minute hand 303 may be positioned on the display unit 251.

The hour hand 301 and the minute hand 303 may move to represent current time in the analog clock mode.

In FIG. 4A, the control unit 180 may receive a setting input to switch the clock mode of the watch-type mobile terminal 100 from the analog clock mode to the digital clock mode.

The control unit 180 may display a digital clock screen 310 including an hour and a minute expressed in digital number on the display unit 251 as illustrated in FIG. 4B according to the received setting input.

Simultaneously, the control unit 180 may control the movements of the hour hand 301 and the minute hand 303 such that the hour hand 301 and the minute hand 303 positioned on the display unit 251 are overlapped with each other.

In this case, the overlapped hand hour 301 and minute hand 303 may be moved to represent the second of current time.

The hour and the minute may be expressed in digital number on the digital clock screen 310 and the second may be expressed by the overlapped hour hand 301 and minute hand 303.

In case that the clock mode of the watch-type mobile terminal 100 operates to the digital clock mode, if the hour hand 301 and the minute hand 303 are moved to express current time, the current time expressed by the hour hand and the minute hand is overlapped with the current time expressed in digital number. In other words, the current time may be meaninglessly repeatedly expressed.

According to an embodiment of the present disclosure, in the digital clock mode, the overlapped hour hand 301 and minute hand 303 are utilized as the second hand, so the hour hand 301 and the minute hand 303 may be effectively used.

In addition, as the hour hand 301 and the minute hand 303 are overlapped with each other, a hidden part is reduced so that the information to be displayed on the display unit 251 is more expanded.

Hereinafter, the operating method of the watch-type mobile terminal 100 according to another embodiment of the present disclosure will be described.

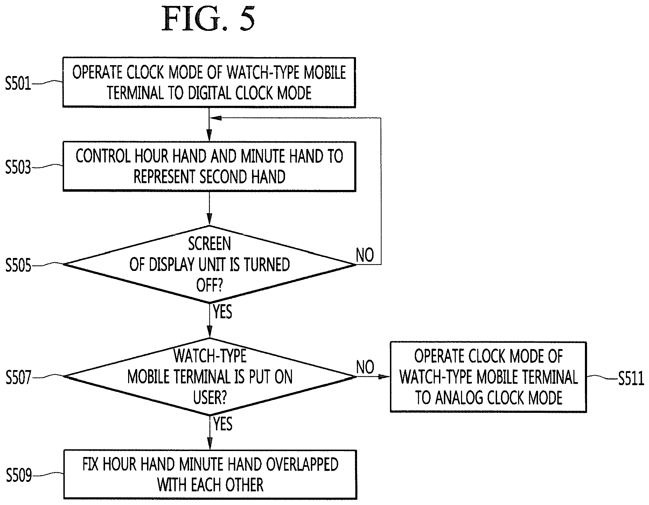

FIG. 5 is a flowchart illustrating the operating method of the watch-type mobile terminal according to another embodiment of the present invention.

The control unit 180 operates the clock mode of the watch-type mobile terminal 100 to the digital clock mode (S501).

The display unit 251 may display digital time and additional information in the digital clock mode.

According to an embodiment, the additional information may include at least one of workout information (e.g., real-time calorie consumption) of a user, year/month/date, schedule information, and notification in formation.

The control unit 180 controls the movements of the hour hand and the minute hand such that the hour hand and the minute hand represent the second hand as the watch-type mobile terminal 100 operates in the digital clock mode (S503).

When the watch-type mobile terminal 100 enters into the digital clock mode, the control unit 240 may control the movements of the hour hand and the minute hand such that the hour hand and the minute hand representing current time and minute are overlapped with each other. Simultaneously, the control unit 240 may control the movements of the hour hand and the minute hand such that the overlapped hour hand and minute hand serve as the second hand.

Thereafter, the control unit 180 determines whether the screen of the display unit 251 is turned off (S505).

That the screen of the display unit 511 is turned off may refer to that any information is not displayed on the screen as the display unit 251 is powered off.

When the screen of the display unit 251 is turned off, the control unit 180 determines whether the watch-type mobile terminal 100 is put on a user (S507).

According to an embodiment, a heartbeat sensor may be provided on the rear surface of the body 201 of the watch-type mobile terminal 100. The heartbeat sensor may sense the heartbeat of the user when the user puts the watch-type mobile terminal 100 on the wrist of the user.

The control unit 180 may determine whether the user puts on the watch-type mobile terminal 100, based on the signal sensed through the heartbeat sensor.

The control unit 180 may determine the watch-type mobile terminal 100 to be put on the user when the pattern of the sensed signal has a preset pattern.

The control unit 180 may determine the watch-type mobile terminal 100 not to be put on the user when the pattern of the sensed signal has no preset pattern.

The control unit 180 may fix the hour hand and the minute hand when the watch-type mobile terminal 100 is put on the user (S509).

In other words, the control unit 180 may fix the overlapped hour hand and minute hand when the watch-type mobile terminal 100 is in put on the user and when the screen of the display unit 251 is turned off.

According to an embodiment, the control unit 180 may control the movements of the hour hand and the minute hand such that the overlapped hour hand and minute hand represents 12'oclock when the watch-type mobile terminal 100 is put on the user and when the screen of the display unit 251 is turned off.

The control unit 180 may fix the hour hand and the minute hand such that the overlapped hour hand and minute hand represent 12'oclock.

When the watch-type mobile terminal 100 is not put on, the control unit 180 may operate the clock mode of the watch-type mobile terminal 100 to the analog clock mode (S511).

In other words, when the watch-type mobile terminal 100 is not put on the user and when the screen of the display unit 251 is turned off, the control unit 180 may operate the clock mode of the watch-type mobile terminal 100 to the analog clock mode.

The control unit 180 may control the movements of the hour hand and the minute hand to represent current time.

The embodiment of FIG. 5 will be described with reference to the following drawings.

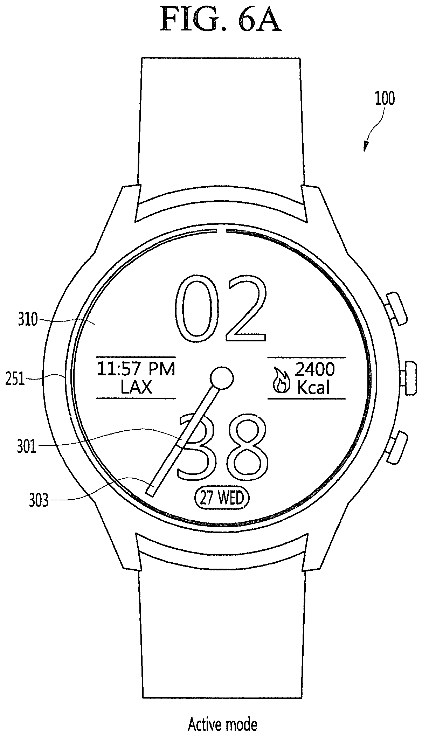

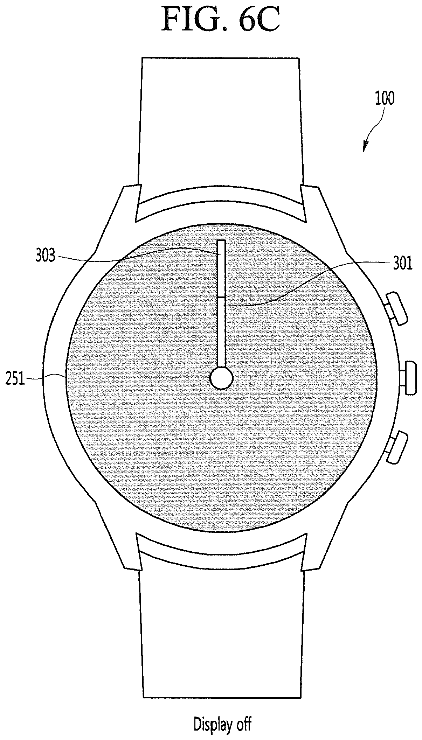

FIGS. 6A to 6C are views illustrating that the movements of the hour hand and the minute hand is controlled as the screen of the display unit 251 is turned off, when the watch-type mobile terminal 100 is put on the user, according to an embodiment of the present disclosure.

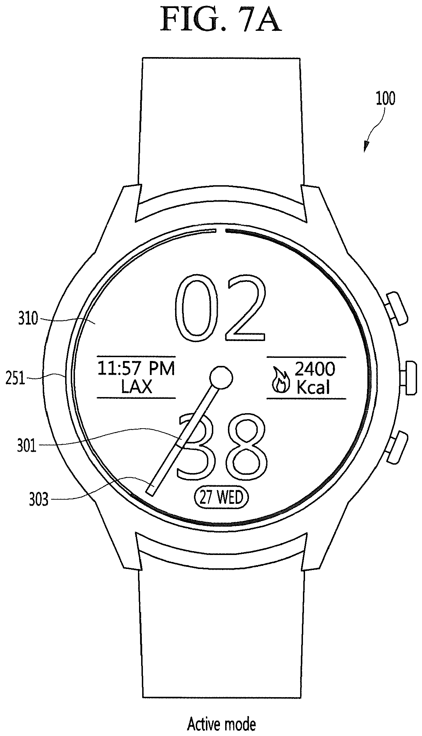

FIGS. 7A to 7C are views that the movements of the hour hand and the minute hand is controlled as the screen of the display unit 251 is turned off, when the watch-type mobile terminal 100 is not put on the user, according to an embodiment of the present disclosure.

Further, in FIGS. 6A to 7C, it is assumed that the operation mode of the display unit 251 may include an active mode, an ambient mode, and an off mode.

The active mode may be a mode that the display unit 251 operates as power having specific intensity or more is supplied to the display unit 251.

The ambient mode may represent a standby state that the minimum power is applied to the display unit 251.

The off mode may represent the state that the screen is turned off as the display unit 251 is not powered.

In addition, the following descriptions will be made on the assumption that the watch-type mobile terminal 100 is put on the user in FIGS. 6A to 6C and is not put on the user in FIGS. 7A to 7B.

The case that the watch-type mobile terminal 100 is not put on the wrist of the user will be described later with reference FIGS. 7A to 7B.

First, FIG. 6A illustrates that the watch-type mobile terminal 100 operates in the digital clock mode in the state that the watch-type mobile terminal 100 is put on the user.

In addition, in FIG. 6A, the display unit 251 is an active mode.

The control unit 180 may control the movements of the hour hand 301 and the minute hand 303 such that the overlapped hour hand 301 and minute hand 303 serve as the second hand.

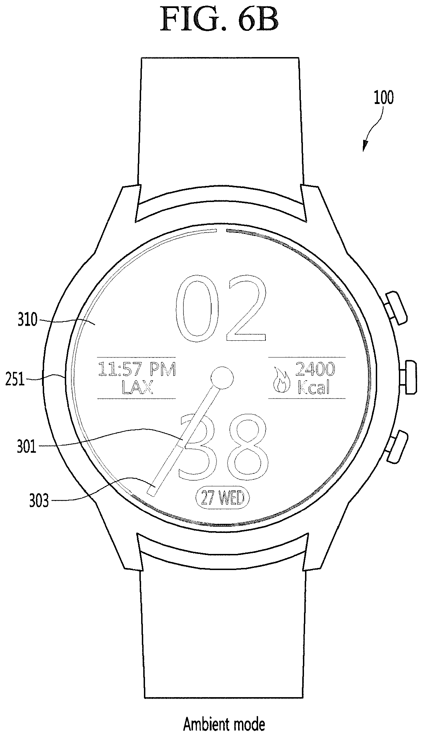

In FIG. 6B, the display unit 251 is in the ambient mode. The control unit 180 may switch the active mode of the display unit 251 to the ambient mode when the display unit 251 does not receive any input for a specific time in the active mode.

In the ambient mode, the display unit 251 may reduce the brightness of a screen as compared with that the display unit 251 is in the active mode. In the ambient mode, the hour hand 301 and the minute hand 303 may perform the second hand.

In the ambient mode, when predetermined time elapses without the reception of any input, the display unit 251 may operate in a screen-off mode.

The control unit 180 may move the hour hand 301 and the minute hand 303 representing 12'oclock when the display unit 251 operates in the screen-off mode. Thereafter, the control unit 180 may fix the hour hand 301 and the minute hand 303. In this case, 12'oclock is provided only for the illustrative purpose, and the movements of the hour hand 301 and the minute hand 303 may be controlled to be fixed at another o'clock.

In other words, the control unit 180 may not apply power to the motor for the movements of the hour hand 301 and the minute hand 303.

When the hour hand 301 and the minute hand 303 are fixed in the state that the screen of the display unit 251 is fixed, the waste of the battery may be reduced.

When the operation mode of the display unit 251 is switched from the off mode to the active mode, or the screen is turned on, the control unit 180 may displays the screen of the digital clock and may control the movements of the hour hand 301 and the minute hand 303 such that the overlapped hour hand 301 and minute hand 303 serve as the second hand.

According to another embodiment, when the operation mode of the display unit 251 is switched to the active mode and the watch-type mobile terminal 100 enters the analog clock mode, the control unit 180 may move the fixed hour hand 301 and minute hand 303 to represent current time.

Hereinafter, the description will be made with reference to FIGS. 7A to 7C.

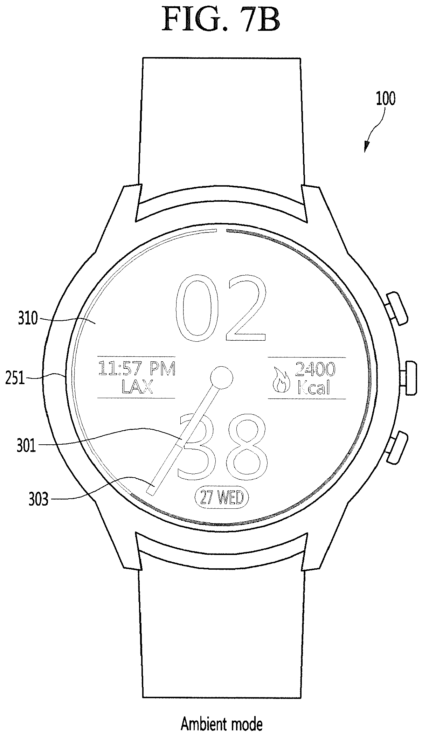

FIGS. 7A and 7B are the same as FIGS. 6A and 6B, the description to be made with reference to FIGS. 7A and 7B will be substituted with the description made with reference to FIGS. 6A and 6B.

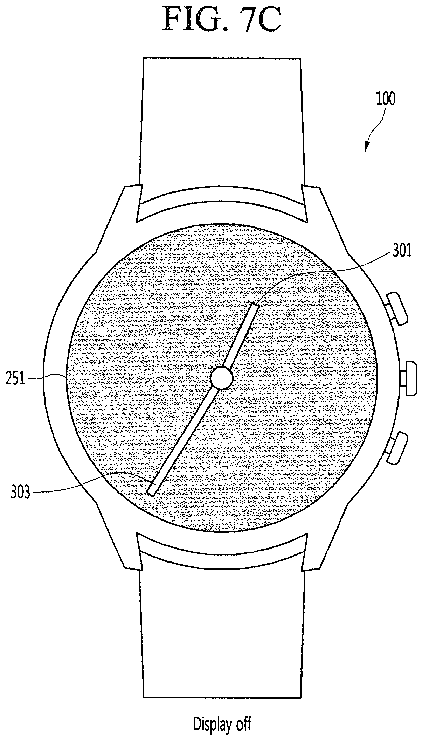

Referring to FIG. 7C, the control unit 180 may control the movements of the hour hand 301 and the minute hand 303 to represent current time when the watch-type mobile terminal 100 is not put on the user and the display unit 251 operates in the off mode.

The control unit 180 may control the movements of the hour hand 301 and the minute hand 303 such that the current time is updated at the time interval of 1 min. Accordingly, the effect of an analog table clock may be obtained when the display unit 251 is in the off mode.

When the operation mode of the display unit 251 is switched from the off mode to the active mode, or the screen is turned on, the control unit 180 may displays the digital clock and may control the movements of the hour hand 301 and the minute hand 303 such that the overlapped hour hand 301 and minute hand 303 serve as the second hand.

The present invention as described earlier may be implemented as a computer readable code in a medium having a program thereon. The computer readable medium includes all kinds of storage devices storing data that may be read by a computer system. Examples of a computer readable medium are a hard disk drive (HDD), a solid state disk (SSD), a silicon disk drive (SDD), a ROM, a RAM, a CD-ROM, a magnetic tape, a floppy disk, and an optical data storage device, and it is also implemented in the form of a carrier wave (e.g., data transmission through the Internet). Also, the computer may also include a control unit 180 of a terminal. Thus, the detailed description should not be construed as limitative in all aspects and should be considered exemplary. The scope of the prevent invention should be defined by the reasonable understanding of the following claims and all changes falling within the equivalent scope of the prevent invention are included in the scope of the prevent invention.

## Claims

1. A watch-type mobile terminal (100) comprising:
a display unit (251);
a physical hour hand;
a physical minute hand;
a control unit (180); and
a computer-readable medium coupled to the control unit (180) having stored thereon instructions which, when executed by the control unit (180), causes the control unit (180) to perform operations comprising:
operating the watch-type mobile terminal (100) in an analog clock mode by displaying, through the hour hand and the minute hand, an hours component and a minutes component of a current time;
receiving a digital mode request configured to switch an operation mode of the watch-type mobile terminal (100) into a digital clock **characterized by**:
based on receipt of the digital mode request, displaying, through the display unit (251), a numerical representation of the hours component and the minutes component of the current time; and
displaying a seconds component of the current time by controlling both the hour hand and the minute hand such that the physical hour hand and the physical minute hand positioned on the display unit (251) are overlapped with each other to indicate the seconds component of the current time.

2. The watch-type mobile terminal (100) of claim 1, wherein the operations comprise:
while operating in the digital clock mode, displaying, through the display unit (251), at least one of a date, a notification, or workout information of a user of the watch-type mobile terminal (100).

3. The watch-type mobile terminal (100) of claim 1 or 2, further comprising a heartbeat sensor configured to sense a heartbeat of a user of the watch-type mobile terminal (100),
wherein the operations comprise:
obtaining a signal through the heartbeat sensor; and
based on the obtained signal, determining a wearing state of the watch-type mobile terminal (100).

4. The watch-type mobile terminal (100) of claim 3, wherein determining the wearing state of the watch-type mobile terminal (100) comprises:
determining whether the obtained signal matches a preset pattern;
based on a determination that the obtained signal matches the preset pattern, determining that the watch-type mobile terminal (100) is worn by the user; and
based on a determination that the obtained signal does not match the preset pattern, determining that the watch-type mobile terminal (100) is not worn by the user.

5. The watch-type mobile terminal (100) of claim 3 or 4, wherein the operations comprise:
based on the wearing state, determining that the watch-type mobile terminal (100) is worn by the user;
determining that a screen of the display unit (251) is in an off-state; and
based on (i) the determination that the watch-type mobile terminal (100) is worn by the user, and (ii) the determination that that the screen of the display unit (251) is in the off-state, stopping display of the seconds component of the current time by stopping respective movements of both the physical hour hand and the physical minute hand.

6. The watch-type mobile terminal (100) of claim 5, wherein the operations comprise:
determining that the screen of the display unit (251) is in an on-state; and
based on (i) the determination that the watch-type mobile terminal (100) is worn by the user, and (ii) the determination that the screen of the display unit (251) is in the on-state, resuming display of the seconds component of the current time by starting respective movements of both the physical hour hand and the physical minute hand.

7. The watch-type mobile terminal (100) of claim 5 or 6, wherein the operations comprise:
receiving an analog mode request configured to switch the operation mode of the watch-type mobile terminal (100) into the analog clock mode;
determining that the screen of the display unit (251) is in an on-state; and
based on (i) receipt of the analog mode request, and (ii) the determination that the screen of the display unit (251) is in the on-state, displaying, through the physical hour hand and the physical minute hand, the hours component and the minutes component of the current time.

8. The watch-type mobile terminal (100) of claim 3 or 4, wherein the operations comprise:
based on the wearing state, determining that the watch-type mobile terminal (100) is not worn by the user;
determining that a screen of the display unit (251) is in an off-state; and
based on (i) the determination that the watch-type mobile terminal (100) is not worn by the user, and (ii) the determination that the screen of the display unit (251) is in the off-state, switching the operation mode of the watch-type mobile terminal (100) into the analog clock mode.

9. The watch-type mobile terminal (100) of claim 8, wherein the operations comprise:
based on the switching of the operation mode of the watch-type mobile terminal (100) into the analog clock mode, displaying, through the physical hour hand and the physical minute hand, the hours component and the minutes component of the current time.

10. The watch-type mobile terminal (100) of claim 9, wherein the operations comprise:
determining that the screen of the display unit (251) is in an on-state; and
based on the determination that the screen of the display unit (251) is in the on-state, displaying the seconds component of the current time by controlling both the physical hour hand and the physical minute hand to indicate the seconds component of the current time.

11. The watch-type mobile terminal (100) of claim 5, wherein stopping the display of the seconds component of the current time comprises:
stopping a supply of power to a motor configured to move the physical hour hand and the physical minute hand.

## Patentansprüche

1. Uhrentyp-Mobilendgerät (100) mit:
einer Anzeigeeinheit (251);
einem physischen Stundenzeiger;
einem physischen Minutenzeiger;
einer Steuereinheit (180); und
einem mit der Steuereinheit (180) gekoppelten computerlesbaren Medium, auf dem Befehle gespeichert sind, die, wenn sie durch die Steuereinheit (180) ausgeführt werden, die Steuereinheit (180) veranlassen, Operationen durchzuführen, die aufweisen:
Betreiben des Uhrentyp-Mobilendgeräts (100) in einem Analoguhrmodus durch Anzeigen durch den Stundenzeiger und den Minutenzeiger einer Stundenkomponente und einer Minutenkomponente einer gegenwärtigen Zeit;
Empfangen einer Digitalmodusanforderung, die konfiguriert ist, einen Betriebsmodus des Uhrentyp-Mobilendgeräts (100) in eine Digitaluhr umzuschalten, **gekennzeichnet durch**:
beruhend auf dem Empfang der Digitalmodusanforderung Anzeigen durch die Anzeigeeinheit (251) einer numerischen Darstellung der Stundenkomponente und der Minutenkomponente der gegenwärtigen Zeit; und
Anzeigen einer Sekundenkomponente der gegenwärtigen Zeit durch Steuern sowohl des Stundenzeigers als auch des Minutenzeigers, so dass der physische Stundenzeiger und der physische Minutenzeiger, die auf der Anzeigeeinheit (251) angeordnet sind, miteinander überlappt sind, um die Sekundenkomponente der gegenwärtigen Zeit anzuzeigen.

2. Uhrentyp-Mobilendgerät (100) nach Anspruch 1, wobei die Operationen aufweisen:
während des Betreibens im Digitaluhrmodus Anzeigen durch die Anzeigeeinheit (251) eines Datums und/oder einer Benachrichtigung und/oder oder Trainingsinformationen eines Benutzers des Uhrentyp-Mobilendgeräts (100).

3. Uhrentyp-Mobilendgerät (100) nach Anspruch 1 oder 2, das ferner einen Herzschlagsensor aufweist, der konfiguriert ist, einen Herzschlag eines Benutzers des Uhrentyp-Mobilendgeräts (100) zu erfassen,
wobei die Operationen aufweisen:
Erhalten eines Signals durch den Herzschlagsensor; und
beruhend auf dem erhaltenen Signal, Feststellen eines Tragezustands des Uhrentyp-Mobilendgeräts (100).

4. Uhrentyp-Mobilendgerät (100) nach Anspruch 3, wobei das Feststellen des Tragezustands des Uhrentyp-Mobilendgeräts (100) aufweist:
Feststellen, ob das erhaltene Signal mit einem voreingestellten Muster übereinstimmt;
beruhend auf einer Feststellung, dass das erhaltene Signal mit dem voreingestellten Muster übereinstimmt, Feststellen, dass das Uhrentyp-Mobilendgerät (100) durch den Benutzer getragen wird; und
beruhend auf einer Feststellung, dass das erhaltene Signal nicht mit dem voreingestellten Muster übereinstimmt, Feststellen, dass das Uhrentyp-Mobilendgerät (100) nicht durch den Benutzer getragen wird.

5. Uhrentyp-Mobilendgerät (100) nach Anspruch 3 oder 4, wobei die Operationen aufweisen:
beruhend auf dem Tragezustand Feststellen, dass das Uhrentyp-Mobilendgerät (100) durch den Benutzer getragen wird;
Feststellen, dass sich ein Bildschirm der Anzeigeeinheit (251) in einem Ausschaltzustand befindet; und
beruhend auf (i) der Feststellung, dass das Uhrentyp-Mobilendgerät (100) durch den Benutzer getragen wird, und (ii) der Feststellung, dass sich der Bildschirm der Anzeigeeinheit (251) im Ausschaltzustand befindet, Stoppen der Anzeige der Sekundenkomponente der gegenwärtigen Zeit durch Stoppen jeweiliger Bewegungen sowohl des physischen Stundenzeigers als auch des physischen Minutenzeigers.

6. Uhrentyp-Mobilendgerät (100) nach Anspruch 5, wobei die Operationen aufweisen:
Feststellen, dass sich der Bildschirm der Anzeigeeinheit (251) in einem Einschaltzustand befindet; und
beruhend auf (i) der Feststellung, dass das Uhrentyp-Mobilendgerät (100) durch den Benutzer getragen wird, und (ii) der Feststellung, dass sich der Bildschirm der Anzeigeeinheit (251) im Einschaltzustand befindet, Wiederaufnehmen der Anzeige der Sekundenkomponente der gegenwärtigen Zeit durch Starten jeweiliger Bewegungen sowohl des physischen Stundenzeigers als auch des physischen Minutenzeigers.

7. Uhrentyp-Mobilendgerät (100) nach Anspruch 5 oder 6, wobei die Operationen aufweisen:
Empfangen einer Analogmodusanforderung, die konfiguriert ist, den Betriebsmodus des Uhrentyp-Mobilendgeräts (100) in den Analoguhrmodus umzuschalten;
Feststellen, dass sich der Bildschirm der Anzeigeeinheit (251) in einem Einschaltzustand befindet; und
beruhend auf (i) dem Empfang der Analogmodusanforderung und (ii) der Feststellung, dass sich der Bildschirm der Anzeigeeinheit (251) im Einschaltzustand befindet, Anzeigen durch den physischen Stundenzeiger und den physischen Minutenzeiger der Stundenkomponente und der Minutenkomponente der gegenwärtigen Zeit.

8. Uhrentyp-Mobilendgerät (100) nach Anspruch 3 oder 4, wobei die Operationen aufweisen:
beruhend auf dem Tragezustand Feststellen, dass das Uhrentyp-Mobilendgerät (100) nicht durch den Benutzer getragen wird;
Feststellen, dass sich ein Bildschirm der Anzeigeeinheit (251) in einem Ausschaltzustand befindet; und
beruhend auf (i) der Feststellung, dass das Uhrentyp-Mobilendgerät (100) nicht durch den Benutzer getragen wird, und (ii) der Feststellung, dass sich der Bildschirm der Anzeigeeinheit (251) im Ausschaltzustand befindet, Umschalten des Betriebsmodus des Uhrentyp-Mobilendgeräts (100) in den Analoguhrmodus.

9. Uhrentyp-Mobilendgerät (100) nach Anspruch 8, wobei die Operationen aufweisen:
beruhend auf dem Umschalten des Betriebsmodus des Uhrentyp-Mobilendgeräts (100) in den Analoguhrmodus Anzeigen, durch den physischen Stundenzeiger und den physischen Minutenzeiger, der Stundenkomponente und der Minutenkomponente der gegenwärtigen Zeit.

10. Uhrentyp-Mobilendgerät (100) nach Anspruch 9, wobei die Operationen aufweisen:
Feststellen, dass sich der Bildschirm der Anzeigeeinheit (251) in einem Einschaltzustand befindet; und
beruhend auf der Feststellung, dass sich der Bildschirm der Anzeigeeinheit (251) im Einschaltzustand befindet, Anzeigen der Sekundenkomponente der gegenwärtigen Zeit durch Steuern sowohl des physischen Stundenzeigers als auch des physischen Minutenzeigers, um die Sekundenkomponente der gegenwärtigen Zeit anzuzeigen.

11. Uhrentyp-Mobilendgerät (100) nach Anspruch 5, wobei das Stoppen der Anzeige der Sekundenkomponente der gegenwärtigen Zeit aufweist:
Stoppen einer Stromzufuhr zu einem Motor, der konfiguriert ist, den physischen Stundenzeiger und den physischen Minutenzeiger zu bewegen.

## Revendications

1. Terminal mobile de type montre (100) comprenant :
une unité d'affichage (251) ;
une aiguille physique des heures ;
une aiguille physique des minutes ;
une unité de commande (180) ; et
un support lisible par ordinateur couplé à l'unité de commande (180) sur lequel sont stockées des instructions qui, lorsqu'elles sont exécutées par l'unité de commande (180), amènent l'unité de commande (180) à mettre en œuvre des opérations comprenant :
faire fonctionner le terminal mobile de type montre (100) dans un mode horloge analogique en affichant, à travers l'aiguille des heures et l'aiguille des minutes, une composante des heures et une composante des minutes d'un temps courant ;
recevoir une demande de mode numérique configurée de manière à commuter un mode de fonctionnement du terminal mobile de type montre (100) sur une horloge numérique, **caractérisé par** :
sur la base de la réception de la demande de mode numérique, afficher, par l'intermédiaire de l'unité d'affichage (251), une représentation numérique de la composante des heures et de la composante des minutes du temps courant ; et
afficher une composante des secondes du temps courant en commandant à la fois l'aiguille des heures et l'aiguille des minutes de sorte que l'aiguille physique des heures et l'aiguille physique des minutes positionnées sur l'unité d'affichage (251) se chevauchent l'une et l'autre pour indiquer la composante des secondes du temps courant.

2. Terminal mobile de type montre (100) selon la revendication 1, dans lequel les opérations comprennent :
tout en fonctionnant en mode horloge numérique, afficher, par l'intermédiaire de l'unité d'affichage (251), au moins l'un des éléments parmi une date, une notification ou des informations d'entraînement d'un utilisateur du terminal mobile de type montre (100).

3. Terminal mobile de type montre (100) selon la revendication 1 ou 2, comprenant en outre un capteur de battement cardiaque configuré de manière à détecter un battement cardiaque d'un utilisateur du terminal mobile de type montre (100) ;
dans lequel les opérations comprennent :
obtenir un signal par le biais du capteur de battement cardiaque ; et
sur la base du signal obtenu, déterminer un état de port du terminal mobile de type montre (100).

4. Terminal mobile de type montre (100) selon la revendication 3, dans lequel l'étape de détermination de l'état de port du terminal mobile de type montre (100) comprend :
déterminer si le signal obtenu correspond à un motif prédéfini ;
sur la base d'une détermination selon laquelle le signal obtenu correspond au motif prédéfini, déterminer que le terminal mobile de type montre (100) est porté par l'utilisateur ; et
sur la base d'une détermination selon laquelle le signal obtenu ne correspond pas au motif prédéfini, déterminer que le terminal mobile de type montre (100) n'est pas porté par l'utilisateur.

5. Terminal mobile de type montre (100) selon la revendication 3 ou 4, dans lequel les opérations comprennent :
sur la base de l'état de port, déterminer que le terminal mobile de type montre (100) est porté par l'utilisateur ;
déterminer qu'un écran de l'unité d'affichage (251) est dans un état désactivé ; et
sur la base (i) de la détermination selon laquelle le terminal mobile de type montre (100) est porté par l'utilisateur, et (ii) de la détermination selon laquelle l'écran de l'unité d'affichage (251) est dans l'état désactivé, interrompre l'affichage de la composante des secondes du temps courant en interrompant les mouvements respectifs de l'aiguille physique des heures et de l'aiguille physique des minutes.

6. Terminal mobile de type montre (100) selon la revendication 5, dans lequel les opérations comprennent :
déterminer que l'écran de l'unité d'affichage (251) est dans un état activé ; et
sur la base (i) de la détermination selon laquelle le terminal mobile de type montre (100) est porté par l'utilisateur, et (ii) de la détermination selon laquelle l'écran de l'unité d'affichage (251) est dans l'état activé, reprendre l'affichage de la composante des secondes du temps courant en démarrant les mouvements respectifs de l'aiguille physique des heures et de l'aiguille physique des minutes.

7. Terminal mobile de type montre (100) selon la revendication 5 ou 6, dans lequel les opérations comprennent :
recevoir une demande de mode analogique configurée de manière à commuter le mode de fonctionnement du terminal mobile de type montre (100) sur le mode horloge analogique ;
déterminer que l'écran de l'unité d'affichage (251) est dans un état activé ; et
sur la base de (i) la réception de la demande de mode analogique, et (ii) de la détermination selon laquelle l'écran de l'unité d'affichage (251) est dans l'état activé, afficher, à travers l'aiguille physique des heures et l'aiguille physique des minutes, la composante des heures et la composante des minutes du temps courant.

8. Terminal mobile de type montre (100) selon la revendication 3 ou 4, dans lequel les opérations comprennent :
sur la base de l'état de port, déterminer que le terminal mobile de type montre (100) n'est pas porté par l'utilisateur ;
déterminer qu'un écran de l'unité d'affichage (251) est dans un état désactivé ; et
sur la base (i) de la détermination selon laquelle le terminal mobile de type montre (100) n'est pas porté par l'utilisateur, et (ii) de la détermination selon laquelle l'écran de l'unité d'affichage (251) est dans l'état désactivé, commuter le mode de fonctionnement du terminal mobile de type montre (100) sur le mode horloge analogique.

9. Terminal mobile de type montre (100) selon la revendication 8, dans lequel les opérations comprennent :
sur la base de la commutation du mode de fonctionnement du terminal mobile de type montre (100) sur le mode horloge analogique, afficher, à travers l'aiguille physique des heures et l'aiguille physique des minutes, la composante des heures et la composante des minutes du temps courant.

10. Terminal mobile de type montre (100) selon la revendication 9, dans lequel les opérations comprennent :
déterminer que l'écran de l'unité d'affichage (251) est dans un état activé ; et
sur la base de la détermination selon laquelle l'écran de l'unité d'affichage (251) est dans l'état activé, afficher la composante des secondes du temps courant en commandant à la fois l'aiguille physique des heures et l'aiguille physique des minutes de manière à indiquer la composante des secondes du temps courant.

11. Terminal mobile de type montre (100) selon la revendication 5, dans lequel l'interruption de l'affichage de la composante des secondes du temps courant comprend :
interrompre une fourniture de puissance à un moteur configuré de manière à déplacer l'aiguille physique des heures et l'aiguille physique des minutes.
